# EUROPEAN PATENT SPECIFICATION

(11) **EP 2 050 409 B1**
(45) Date of publication and mention of the grant of the patent: **29.06.2011**
(21) Application number: 08016131.8
(22) Date of filing: 12.09.2008
(51) Int. Cl.: A61B 18/14

(54) **High frequency tool**
Hochfrequenzwerkzeug
Outil à haute fréquence

(30) Priority: 17.10.2007 JP 2007270029
(43) Date of publication of application: 22.04.2009
(73) Proprietor: FUJIFILM Corporation, Tokyo (JP); Toyonaga, Takashi, Izumi-shi Osaka (JP)
(72) Inventor: Toyonaga, Takashi, Osaka (JP); Kadouno, Yoshinori, Saitama-shi, Saitama (JP); Izaki, Toshihiko, Saitama-shi, Saitama (JP)
(74) Representative: Klunker . Schmitt-Nilson . Hirsch

(56) References cited:
- EP-A- 1 709 924
- EP-A- 1 726 266
- EP-A- 1 752 108
- WO-A-03/024349
- US-A1- 2004 210 215

## Description

### [FIELD OF THE ART]

This invention relates to a high frequency tool according to the preamble of claim 1. It is used in endoscopic electro-surgery, more particularly in a mucosal dissection treatment or the like.

### [TECHNICAL BACKGROUND]

When a lesion like a tumor is spotted in a mucosal layer of an intracavitary wall in the esophagus, stomach, duodenum or large intestine by an endoscopic examination, it becomes necessary to excise the spotted mucosal lesion. The excision can be made, for example, by a treatment generally referred to as "Endoscopic Submucosal Dissection (ESD)." An ESD operation is conducted in the manner as follows. In the first place, a mucosal lesion, i.e., a target of excision, is demarcated with markings and then swelled and elevated by a regional injection. In this state, the mucosal lesion is dissected along demarcation markings by the use of a high frequency surgical instrument, incising fibrous tissues in a submucosal layer to peel off a mucosal lesion from a muscle layer.

Generally, a high frequency surgical instrument to be used in an ESD operation consists of a rod-like electrode member which is put in a flexible sheath to serve as a high frequency knife. A manipulation means is connected to a proximal end of the flexible sheath thereby to protrude the high frequency knife out of the sheath. A high frequency current is conducted through the knife at the time of a mucosal dissection.

In this connection, knives for use in ESD treatments can be categorized into an acicular knife employing a straight electrode member, e.g., as described in Patent Literature 1 (Japanese Laid-Open Patent Application 2007-68596), and a hook type knife employing a rod-like electrode member with a large diameter or L-shaped hook portion at a distal end, e.g., as described in Patent Literature 2 (Japanese Laid-Open Patent Application 2004-313537. An acicular knife is best suited for piercing a mucous membrane, and is capable of mucosal dissections or excisions by putting the electrode member in a horizontal movement or swinging movement. On the other hand, in the case of a hook type knife, a mucous membrane is caught by a hook portion at the distal end of the knife, and dissected or excised by manipulating the hook portions in such a way as to pull in the mucous membrane.

### [PROBLEM(S) TO BE SOLVED BY THE INVENTION]

Desirably, a high frequency surgical instrument to be used in an ESD treatment should have not only functions of putting demarcation markings on the surface mucosa and excising a mucosal legion by dissection of a mucosal membrane and a submucosal fibrous layer but also a function of stanching bleeding by a coagulative cauterization treatment, which is usually required in an ESD treatment. Namely, it is desirable that all of these surgical treatments can be performed by the use of a single surgical instrument. In this regard, it may be possible to give all of these surgical treatments by the use the high frequency surgical instrument of Patent Literature 1 mentioned above.

However, the high frequency surgical instrument of Patent Literature 1 which employs an acicular electrode is capable of dissecting or excising a mucous membrane smoothly but is not necessarily satisfactory in performance as a marking instrument or as a cauterization instrument for a coagulative stanching treatment because the electrode member is narrowed down toward its distal end. In contrast, the high frequency instrument of Patent Literature 2, employing an electrode member having a circular or triangular plate-like electrode portion at its distal end, is suitably used in marking and a coagulative stanching treatment but is not suited for use in dissecting a mucous membrane because it is terminated with a flat plate-like portion at its distal end. Besides, at the time of excision of a mucous membrane, the electrode member is manipulated in such a way as to hook up fibrous tissues in a submucosal layer, so that there are possibilities of damaging healthy tissues with edges on the electrode member when the latter is put in an incising action. Further, the existence of edges on the electrode member makes it difficult to conduct current in a uniform density through the entire plate-like electrode portion, resulting in failures in giving a uniform cauterization treatment in some cases.

In accordance with the preamble of claim 1, EP 1 752 108 A discloses a highfrequency tool having a rod-shaped electrode member the front end of which is formed semi-spherically. A similar tool is known from EP 1 726 266.

WO 03/024349 A1 discloses a tool having an electrode member comprising a spherical end surface portion and a cylindrical side surface portion contiguous to the spherical end surface portion.

### [DISCLOSURE OF THE INVENTION]

With the foregoing situations in view, it is an object of the present invention to provide a high frequency tool for use in endoscopic electro-surgery, more particularly, a high frequency tool which is capable of performing a series of surgical treatments such as marking of a mucosal lesion, mucosal dissection and excision and stanching by a coagulative cauterization treatment.

### [MEANS FOR SOLVING PROBLEMS]

In order to solve the above-stated objective, according to the present invention, there is provided a high frequency tool having the features of claim 1.

In this instance, the electrode member which makes up the high frequency knife is at least provided with a rod portion and a spherically bulged portion which is formed contiguously at a fore distal end of the rod portion. Both of the rod portion and spherically bulged portion are formed of an electrically conductive metallic material like stainless steel. The rod portion and the spherically bulged portion may be formed as separate parts and connected with each other afterwards by welding or other suitable means. However, considering the necessity for a surface smoothing treatment prior to a welding operation, it is desirable to form these parts of the electrode member on one integral structure by machining. The spherically bulged portion may be formed in a spherical shape including transitional boundary regions to the rod portion. Alternatively, the spherically bulged portion may be formed in a spherical shape on the front side and in a moderately curved shape on the rear side including transitional regions to the rod portion.

A flexible cord is connected to the electrode member for power supply. By pushing or pulling the flexible power supply cord within the flexible sheath, the electrode member is caused to protrude from or retract into a fore distal end of the flexible sheath. A tubular rigid tip member is fitted in a fore distal end portion of a body tube of the flexible sheath. A fore end face of the rigid tip member may be projected from a fore distal end face of the body tube of the flexible sheath, or may be disposed flush with the fore distal end face of the body tube to form a flat annular end face of a relatively large width at the fore distal end of the flexible sheath. Attached to a proximal end of the flexible sheath is a manipulation means which is composed of a shank portion, e.g., including a connector pipe member which is connected to the flexible sheath, and a slider which is slidably fitted on the shank portion for sliding movements in the axial direction of the shank portion. By way of a liquid feed means which is connected to the connector pipe member, a liquid can be sent into a body cavity through the high frequency tool, for example, for washing away blood or other contaminants from a site of electro-surgery within a body cavity.

For a liquid supply from the above-mentioned liquid feed means, there may be provided a liquid supply passage separately from the body tube accommodating the electrode member and the flexible cord. Alternatively, the axial through hole of the rigid tip member can be utilized as a liquid supply passage. In order to make it possible to supply a liquid even when the electrode member is projected out of the flexible sheath, preferably axial ridge portions are provided on the rod portion of the electrode member, at angular intervals around the circumference of a rod portion on a proximal side away from the spherically bulged portion. The respective ridge portions stand to an inscribing height relative to the axial passage hole of the rigid tip member to define communication passages between adjacent ridge portions within the axial passage hole. At a proximal end, each ridge portion is provided with a radial projection which is adapted to be brought into abutting engagement with a proximal end face of the rigid tip member to delimit a maximum protrusion length of the electrode member from the fore distal end of the flexible sheath. Thus, a liquid can be supplied to a body cavity through the communication passages which are defined between adjacent ridge portions on the rod portion of the electrode member to intercommunicate inside and outside of the flexible sheath. On the other hand, when the electrode member is retracted into the flexible sheath, the spherically bulged portion at the fore distal end of the electrode member can be located in a position on the proximal side of the rigid tip member, permitting to use the entire sectional area of the axial passage area as a liquid supply passage.

The spherically bulged portion is formed in a spherical shape at least on the front side so that it can be brought into abutting engagement with surface mucosa over a broad surface area and with a uniform surface pressure. Therefore, there is little possibility of uneven cauterization at the time of cauterizing mucosa for marking or stanching purposes, that is to say, the tool is capable of uniform cauterization over broad surface areas of mucosa. Besides, at the time of stabbing the electrode member into mucosa for a dissection treatment, it may be difficult to manipulate the electrode member smoothly as compared with an acicular electrode. However, since the fore distal end of the electrode member is formed in a round spherical shape instead of a flat shape, it can be driven into the mucosa without difficulty. In addition, the fore distal end of the flexible sheath is terminated with a flat annular surface of a relatively large breadth, which is formed jointly by end faces of the body tube of the flexible sheath and the tubular rigid tip member. Therefore, when incising a mucosal layer after stabbing same with the electrode member, the rod portion of the electrode member functions as an incision knife. At this time, the mucosal layer is securely gripped between the spherically bulged end of the electrode member and the flat end face which is formed jointly by the flexible sheath and the rigid tip member, keeping the electrode member in a stable state and preventing its deviational movements during a dissection treatment. Further, a mucosal layer can be excised by swinging back and forth the electrode member which is led out of the flexible sheath. Since the electrode member is formed in an edgeless shape, it is free from such problems like uneven distribution of current density and destruction of healthy mucosa.

In order to let the high frequency tool perform the demarcation marking of surface mucosa, dissection and excision treatments and hemostatic cauterization smoothly without giving damages to a muscle layer which exists beneath a submucosal layer, it is preferable for the tool to have a protrusion length of 0.5mm to 4mm from a fore distal end of the tubular rigid tip member, a diameter of 0.4mm to 0.6mm in said rod portion, and a maximum outside diameter of 0.8mm to 1.2mm in said spherically bulged portion.

The above and other objects, features and advantages of the present invention will become apparent from the following particular description of the invention, taken in conjunction with the accompanying drawings which show by way of example preferred embodiments of the invention. Needless to say, the present invention is not limited to particular forms shown in the drawings.

### [BRIEF DESCRIPTION OF THE DAWINGS]

In the accompanying drawings:
Fig. 1 is a schematic illustration showing general construction of a high frequency tool according to the present invention;
Fig 2 is a partly cutaway longitudinal section, showing essential parts of the high frequency tool of Fig. 1 on an enlarged scale;
Fig. 3 is a fragmentary longitudinal section showing a fore end portion of the high frequency tool on an enlarged scale;
Fig. 4 is a cross-sectional view taken on line A-A of Fig. 3;
Fig. 5 is a fragmentary longitudinal section similar to Fig. 3, showing a high frequency knife which is retracted rearward of a guide member;

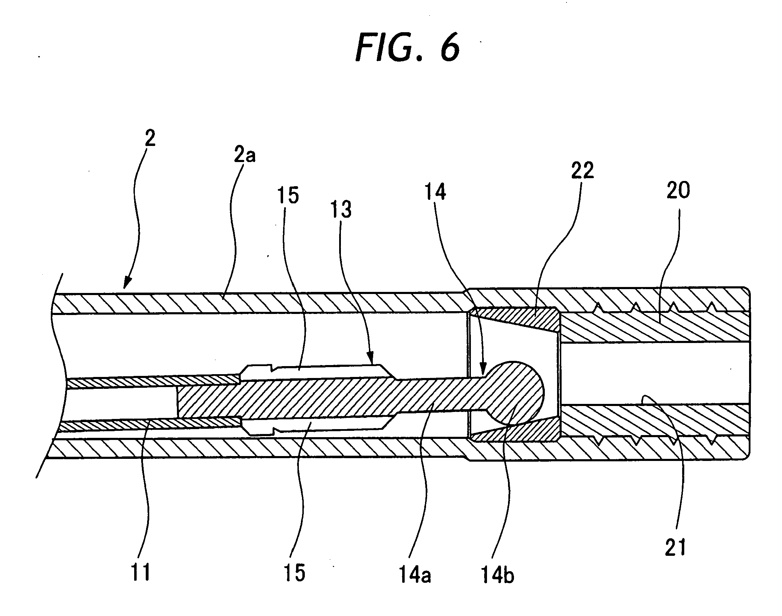

Fig. 6 is a fragmentary longitudinal section similar to Fig. 3, showing a rod-like electrode of the high frequency knife which is being guided by the guide member;
Fig. 7 is a schematic plan view of the high frequency knife;
Fig. 8 is a schematic front view of the high frequency knife;
Fig. 9 is a fragmentary perspective view of the high frequency tool according to the invention, which is being manipulated to protrude forward from an instrument insertion channel of an endoscope;
Fig. 10 is a schematic plan view of a mucosal lesion demarcated by circumventive markings;
Fig. 11 is a schematic view of the high frequency tool in use for putting on markings on surface mucosa;

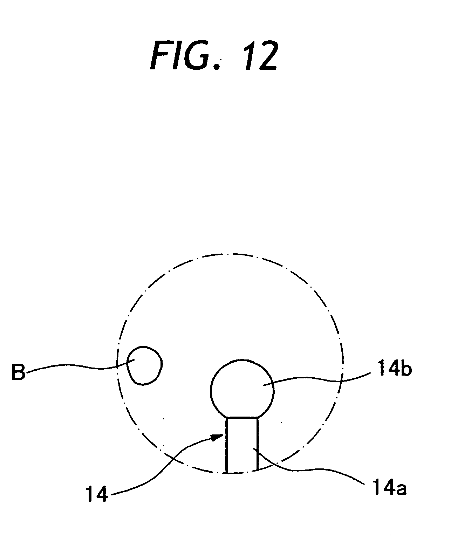

Fig. 12 is a schematic view of the high frequency tool being projected out of an endoscopic instrument insertion channel, taken through an endoscopic observation window;
Fig. 13 is a schematic sectional view of a mucosal lesion receiving a regional injection;
Fig. 14 is a schematic sectional view of the mucosal lesion being pierced with a high frequency knife of the high frequency tool;
Fig. 15 is a schematic sectional of the mucosal lesion in a mucosal dissection treatment;
Fig. 16 is a schematic plan view of the mucosal lesion including a diseased mucosal region dissected by the high frequency instrument;
Fig. 17 is a schematic sectional view of the mucosal lesion in a stage of a mucosal excision treatment;
Fig. 18 is a schematic sectional view of the high frequency tool in a stage of washing away blood and other body fluids from a bulged spherical portion at the fore end of the electrode member; and
Fig. 19 is a schematic sectional view of a high frequency tool in another embodiment of the invention.

### [BEST MODE FOR CARRYING OUT THE INVENTION]

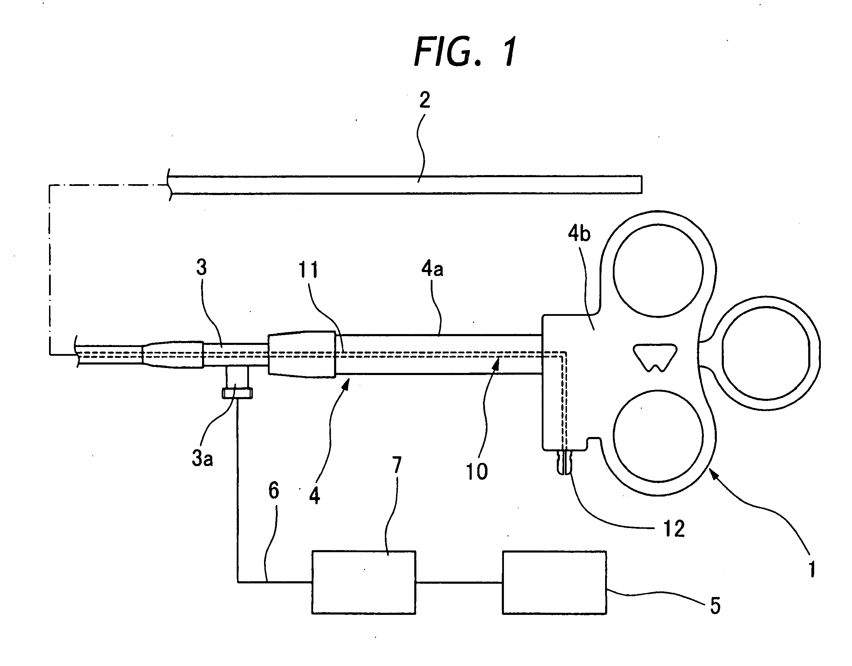

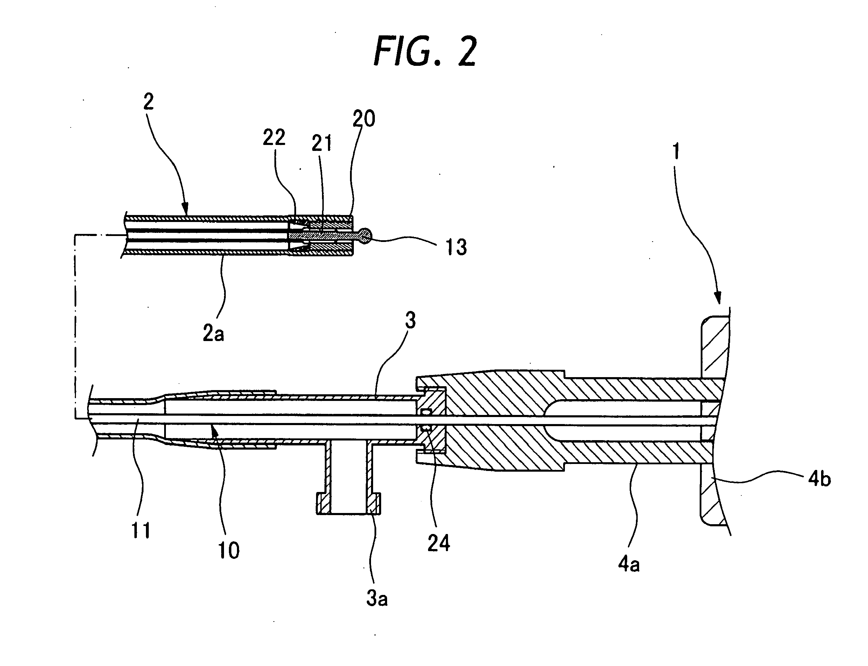

Hereafter, the invention is described more particularly by way of its preferred embodiments shown in the accompanying drawings, of which Fig. 1 shows the general construction of a high frequency tool according to the invention and Fig. 2 shows its essential parts in section and on an enlarged scale.

In Figs. 1 and 2, indicated at 1 is a high frequency tool, which is composed of an elongated flexible sheath 2, a connector pipe 3 attached to the proximal end of the sheath 2 at its fore end, and a manipulation means 4 attached to the other proximal end of the connector pipe 3. In turn, the manipulation means 4 is composed of a shank portion 4a which is joined with the connector pipe 3 at its fore end, and a slider member 4b which is slidably fitted on the shank portion 4a for sliding movements in the axial direction. Connected to the slider member 4b is a proximal end of a flexible cord 11 which constitutes a main core structure 10 of the instrument.

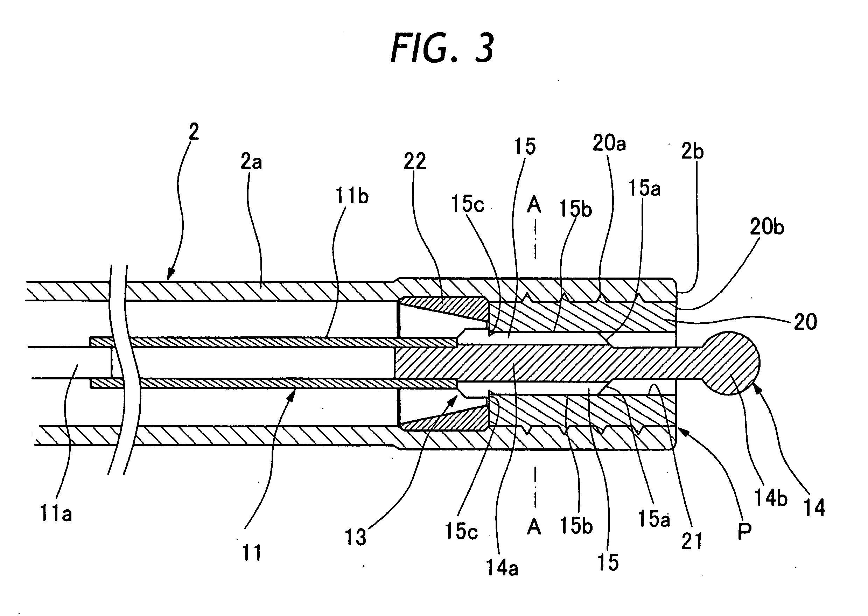

As shown in Fig. 3, the flexible cord 11 is composed of a solid conducting wire portion 11a and a hollow conducting wire portion 11b which is connected to a fore end of the solid conducting wire portion 11a. Both of the solid and hollow wire portions 11a and 11b have the respective exterior surfaces covered with an insulating coating. Further, these solid and hollow wire portions 11a and 11b are flexible at least in bending directions. A proximal end portion of the solid wire portion 11a is projected out of the slider member 4b to provide a contact portion 12. This contact portion12 is disconnectibly connectible to a high frequency power source which is not shown.

The flexible cord 11, a major constituent part of the main core structure 10 of the high frequency tool, is extended forward through the flexible sheath 2 from a proximal end of the connector pipe 3 which is connected to the slider member 4b, the fore end of the flexible cord 11 being connected to a high frequency knife 13 which is manipulatable in retracting and protruding directions for retraction or protrusion into or out of the flexible sheath 2. The high frequency knife 13 is provided with an electrode member 14 for conduction of a high frequency current which is supplied by way of the solid conducting wire 11a of the flexible cord 11. The electrode member 14 is composed of a rod portion 14a and a spherically bulged portion 14b which is appreciably larger in diameter than the rod portion 14a. For power supply, the proximal end of the rod portion 14a, that is, the base end of the rod portion 14a which is located on the proximal side away from its fore end, which is connected to the spherically bulged portion 14b, is electrically connected to the solid conducting wire portion 11a of the flexible cord 11 through the hollow wire portion 11b. The spherically bulged end 14b and part of the rod portion 14a are extended out of the flexible sheath 2 over a predetermined protrusion length for contact with tissues to be cauterized or dissected.

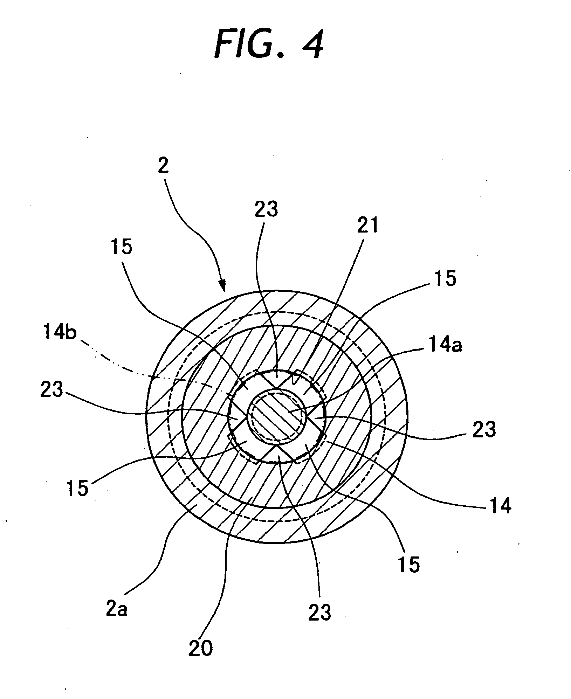

As shown in Figs. 3 and 4, the flexible sheath 2 has a body tube 2a having a tubular rigid tip member 20 fitted and anchored in its fore distal end portion. The tubular rigid tip member 20 is made of an electrically insulating, heat resistant material, preferably, made of a ceramic material. A helical protuberance 20a is formed around the outer periphery of the tubular tip member 20. That is to say, by way of the helical protuberance 20a, the tubular tip member 20 is threaded into a distal end portion of the body tube 2a and anchored in position on the latter. In addition, by way of the helical protuberance 20a, the tip member 20 is restrained of a dislodging movement toward the fore end of the body tube 2a. If desired, the tubular rigid tip member 20 may be fixed in position at the fore end of the body tube 2a by the use of an adhesive agent instead of the threaded fitting engagement as described above.

The tubular rigid tip member 20 is provided with an axial passage hole 21 to project or retract a high frequency knife 13 therethrough. The spherically bulged portion 14a at the distal end of the electrode member 14 of the high frequency knife 13 can pass through the axial passage hole 21 which is so arranged as to have a minimum necessary inside diameter for guaranteeing smooth passage of the spherically bulged end portion 14a of the electrode member 14. The opposite end faces of the rigid tip member 20 are disposed normal to its longitudinal axis. The rigid tip member 20 is threaded into the body tube 2a such that the front end face of the rigid tip member 20 is disposed substantially flush with the fore end face of the body tube 2a. Accordingly, the flexible sheath 2 comes to have an annular end wall P with a flat annular surface.

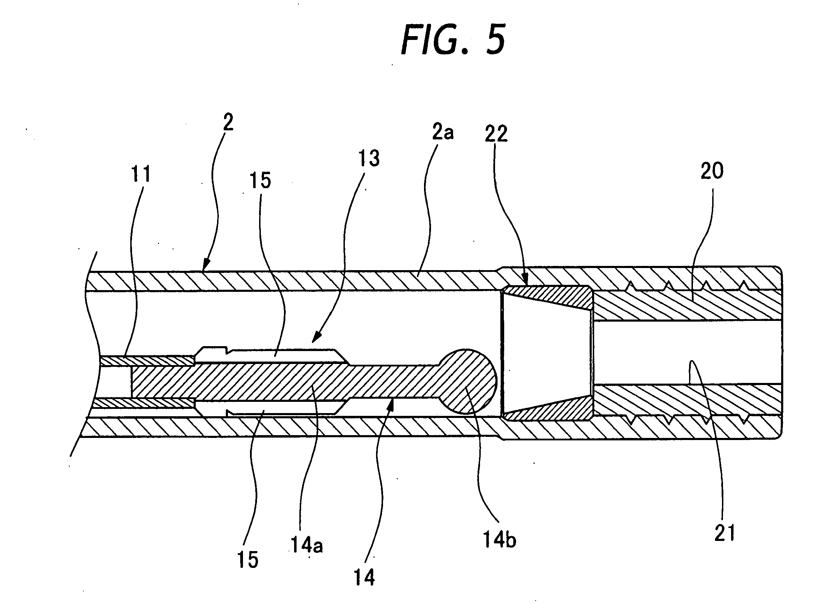

Attached to the proximal end of the rigid tip member 20 is a guide member 22 with a tapered passageway for guiding the high frequency knife 13 securely toward the axial passage hole 21 from a position on the proximal side of the axial passage hole 21. More specifically, the guide member 22 is in the form of a ring which is formed of stainless steel or ceramic material covered with an insulating coating, and which defines a tapered guide way on the inner peripheral side. Normally, the high frequency knife 13 is retracted to a position on the proximal side of the rigid tip member 20 as shown in Fig. 5, leaving the axial passage hole 21 in a completely unoccupied state from rear to fore end. As the flexible cord 11 is pushed forward by way of the manipulation means, the high frequency knife 13 is moved forward toward the axial passage hole 21 along the tapered guide wall of the guide member 22, urging the spherically bulged end 14b of the electrode 14 to advance into the axial passage hole 21 in a guided state.

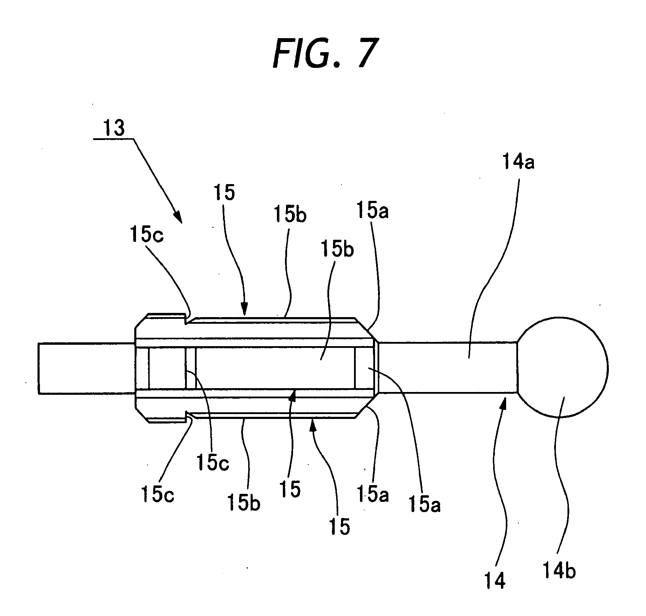

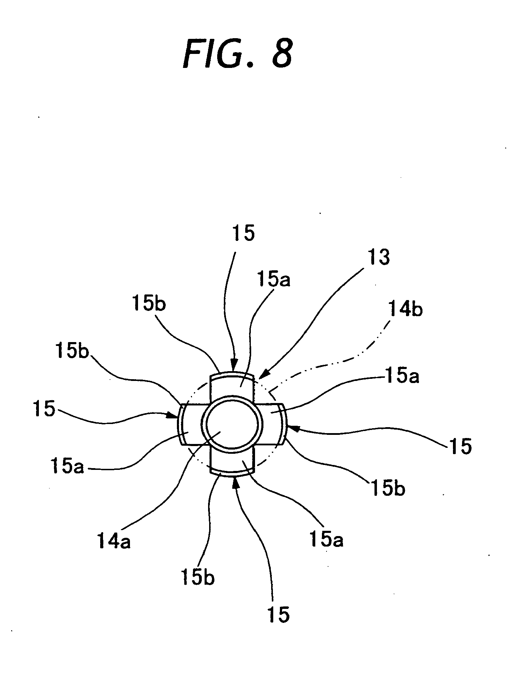

As shown in Figs. 7 and 8, the rod portion 14a of the electrode member 14 of the high frequency knife 13 is in the form of a straight rod of a predetermined length, which is formed of a conducting material and provided with axial ribs or ridges 15 at four equidistant angular positions around the circumference of a proximal end portion. Each axial ridge 15 is provided with an inclined surface 15a at a fore end, which is sloped radially inward in a forward direction to ensure smooth entrance of the high frequency knife 13 into the axial passage hole 21 of the rigid tip member 20. Each axial ridge 15 is extended over a predetermined distance in the axial direction and provided with an outer surface 15a which is curved in the circumferential direction with substantially the same radius of curvature as the inner wall surface of the axial passage hole 21 of the rigid tip member 20. Accordingly, upon entering the axial passage hole 21 of the rigid tip member 20, the outer surfaces 15a of the four axial ridges 15a of the high frequency knife 13 are brought into sliding contact with inner wall surfaces of the axial passage hole 21. As a result, the electrode member 14 with the spherically bulged head portion 14b is automatically brought to a center position in the axial passage hole 21 of the rigid tip member 20 and can be moved smoothly along the center axis of the latter in a stabilized state free of vibratory movements.

Further, in a proximal end portion, each axial ridge 15 is provided with a stepped stopper wall 15c rising in a radially outward direction. The stopper wall 15c on each radial blade 15 is brought into abutting engagement with a rear end face of the tubular rigid tip member 20 to limit a protrusion length of the electrode member 14 through the axial passage hole 21 of the rigid tip member 20. Namely, the stopper walls 15c on the respective radial blades 15 are each located in a position which delimits a maximum protrusion length of the high frequency knife 13 out of the flexible sheath 2. Since the high frequency instrument 1 is used for excision of a mucosal lesion within a body cavity, the maximum protrusion length of the electrode member 14 of the high frequency knife 13 beyond the annular end surface P at the fore distal end of the flexible sheath 2 is set in a length which is larger than the thickness of a mucous membrane on an intracavitary wall but smaller than a sum of thicknesses of the mucous membrane and a submucosal layer.

In this instance, the protrusion length of the electrode member 14 beyond the annular flat end face P at the distal end of the flexible sheath 2 is preferably in the range of 0.5mm to 4mm from the standpoint of safety. Further, the diameter of the rod portion 14a of the electrode member 14 is preferably in the range of 0.4mm to 0.6mm, while the outside diameter of the spherically bulged portion 14b is preferably in the range of 0.8mm to 1.2mm and the axial passage hole 21 has an inside diameter which is slightly larger than the spherically bulged portion 14b.

When the electrode member 14 is protruded to a maximum length, four communication passages 23 are formed between adjacent axial ridge portions 15 as shown in Fig. 4 in a height based on a diametral difference between the rod portion 14a of the electrode member 14 and the axial passage hole 21 of the rigid tip member 20. The connector pipe 3 which is provided at the proximal end of the flexible sheath 2 of the high frequency tool 1 is provided with a tube connection port 3a to receive a supply of a biocompatible liquid such as physiological saline, sodium hyaluronate or the like. As shown in Fig. 1, a liquid feed tube 6 from a storage tank 5 is detachably connectible to the tube connection port 3a of the connector pipe 3. Provided in the course of the liquid feed tube 6 is a switch means 7 like a foot switch thereby to open and close a liquid flow passage in controlling the liquid supply to the connector pipe 3. The flexible sheath 2 internally defines a liquid supply passage in communication with the connector pipe 3 which is attached to the proximal end of the flexible sheath 2. In this instance, the flexible cord 11 which constitutes the main core structure 10 of the instrument 1 is connected to the slider 4b of the manipulation means 4 through the flexible sheath 2 and the connector pipe 3. In the connector pipe 3, a seal member 24 is fitted around the flexible cord 11 to prevent a reverse flow of physiological saline, for example.

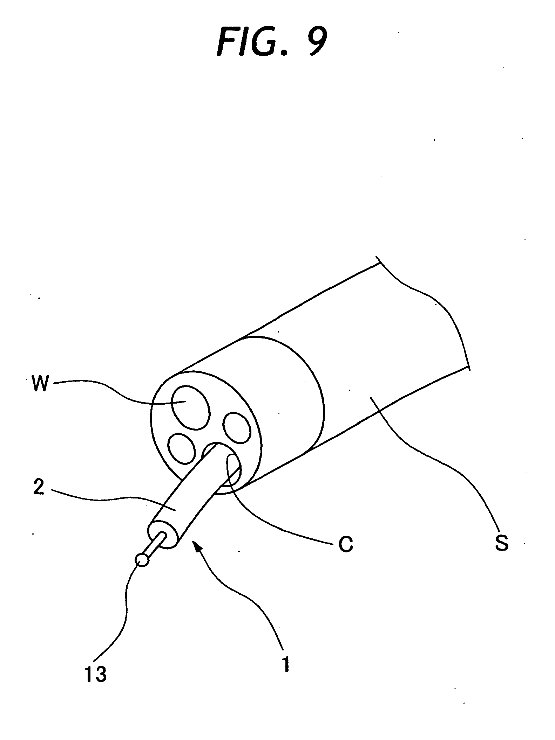

As shown in Fig. 9, the high frequency tool 1 of the above construction is introduced into a body cavity by way of an instrument insertion channel C which is provided internally of an endoscopic insertion rod S in the vicinity of an endoscopic observation window W. In case a mucosal lesion is spotted in an intracavitary wall of the esophagus, duodenum or large intestine, the mucosal lesion is excised by endoscopic surgery. Given below is an example of an endoscopic surgical treatment for excision of a diseased mucosal region the existence of which has been confirmed beforehand by an endoscopic examination.

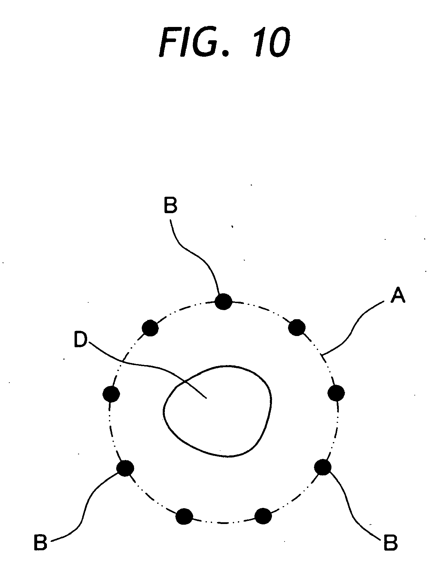

In the first place, as shown in Fig. 10, a diseased mucosal region D is demarcated with circumventive dot-like markings. The markings are limited to a region which is necessary for complete excision of a diseased portion with the least damages to other healthy portions. The markings in the form of cauterization spots B are made at suitable intervals along borders of the diseased mucosal region D by the use of the endoscopic high frequency tool 1, in the manner as follows.

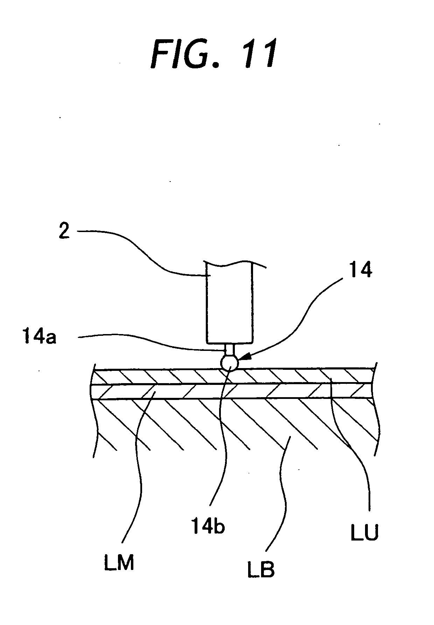

Namely, the high frequency tool 1 is put into an instrument insertion channel C of an endoscopic insertion rod as far as an outlet opening at the fore distal end of the insertion rod, and then the flexible sheath 2 is projected from the outlet opening. In the next place, as shown in Fig. 11, the high frequency knife 13 is led out of the flexible sheath 2 to a position confronting a border portion of the diseased mucosal region D at a predetermined distance. In this state, the fore distal end, that is, the spherical bulged portion 14b of the electrode member 14 is abutted against a surface of a mucous membrane. At this time, the position of the electrode member 14 of the high frequency knife 13 is monitored in a view field through the endoscopic observation window W. In this instance, in an observation image through the endoscopic observation window W, behaviors of the spherically bulged head portion 14b of the electrode member 14 can be observed clearly to demarcate the diseased mucosal region D with markings accurately.

It is the surface mucosal layer LU that is marked with the cauterization spots, i.e., the mucosal layer LU which overlies a submucosal layer LM and a muscle layer LB. As the electrode member 14 is lightly pressed on the mucosal layer LU, the surface of the latter is deformed in a concave shape, bringing the spherically bulged portion 14b of the electrode member 14 into abutting engagement with the mucosal layer LU over a broader surface area. Besides, the spherically bulged portion 14b is pressed against the mucosal layer LU with a uniform force in the entire contacting surface areas, so that, when a high frequency current is applied by the high frequency knife 13, a current conduction of uniform density takes place substantially in the entire contacting areas of the mucosal layer LU, that is to say, the entire contacting areas are uniformly cauterized to bear a clear marking.

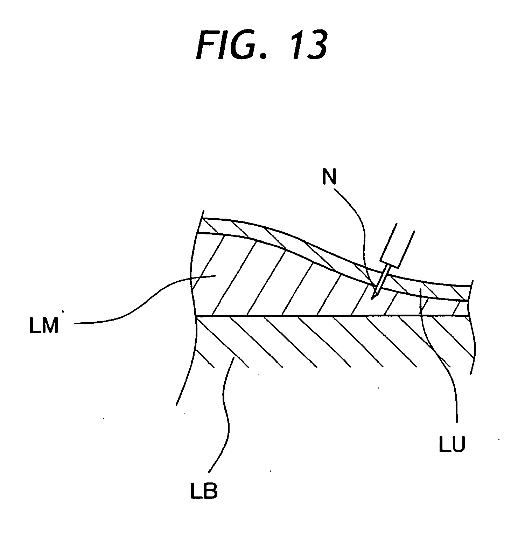

In the next place, as shown in Fig. 13, physiological saline, sodium hyaluronate or the like is shot into the diseased mucosa by a regional injection. For this purpose, the high frequency tool 1 is once drawn out from the endoscopic instrument insertion channel C, and instead a regional injection means N is inserted into the endoscopic instrument insertion channel C to make a regional injection into the submucosal layer LM between the muscle layer LB and the mucosal layer LU to swell and elevate the submucosal layer LM. By swelling and elevating the submucosal layer LM in this manner, the mucosa LU can be set at a greater distance from the muscle layer LB so that it can be treated smoothly in a safer manner.

The circumventive demarcation marking of the diseased mucosal region D is followed by a dissection along the borders of the marked mucosal region D. For this purpose, after swelling and elevating the submucosal layer LM to a sufficient degree, the regional injection means N is extracted from the endoscopic instrument insertion channel C, and instead the high frequency instrument 1 is inserted into the endoscopic instrument insertion channel C again, letting the flexible sheath 2 protrude from the endoscopic insertion rod S. At this time, it is desirable that the whole high frequency knife 13 be still in a retracted position within the flexible sheath 2. However, it would cause no inconvenience in particular if the high frequency knife 13 were projected out of the flexible sheath 2. In this state, the fore distal end of the flexible sheath 2 is brought into contact with a surface of the mucosa in the borders of the diseased mucosal region D. At this time, preferably the annular flat end face P (see Fig. 3), which is formed jointly by the end faces of the body tube 2a of the flexible sheath 2 and the rigid tip member 20, is positioned to confront the mucosal layer LM and then lightly put on a surface of the latter, applying as small a pressing force as possible.

The annular flat end face P needs to have a broad surface area in order to prevent deformations of the mucosal layer LU at the time when the fore distal end of the high frequency instrument 1 is lightly pressed on a surface of the mucosal layer LU. Formed inside the annular flat end face P are the communication passages 23 which have passage areas slightly larger than the diameter of the spherically bulged portion 14b of the electrode member 14. Based on this condition, the surface area of the annular flat end face P should preferably be broadened as much as possible. By so arranging the annular flat end face P, the surface of the mucosal layer LU is deformed only in a least degree when the high frequency instrument 1 is pressed thereagainst, keeping a sufficient distance to the muscle layer LB.

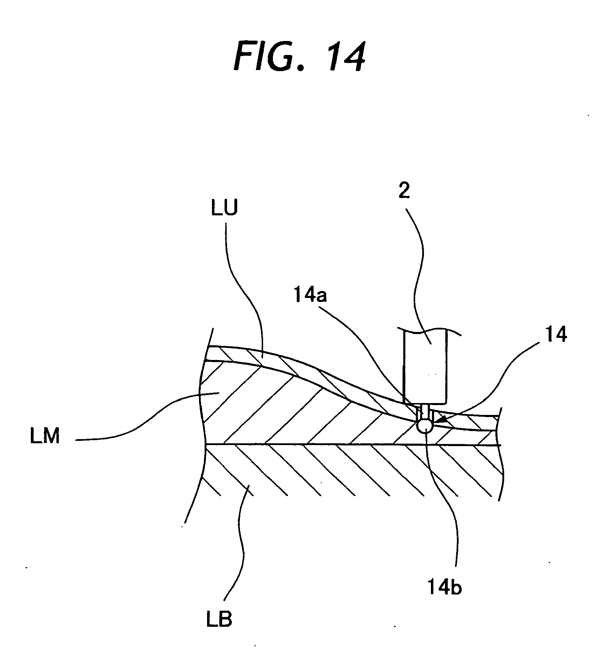

While holding the annular flat end face P, i.e., the combination of the end faces 2b and 20b of the body tube 2a and the rigid tip member 20, in abutting engagement with the mucosal layer LU, the electrode member 14 of the high frequency knife 13 is projected out of the flexible sheath 2 by pushing the manipulation means 4 forward. In case the electrode member 14 is already projected beforehand, the flexible sheath 2 is pushed gradually toward the mucosal layer LU, and a high frequency current is conducted through the electrode member 14. Whereupon, as shown in Fig. 14, the spherically bulged portion 14b at the fore distal end of the electrode member 14 is urged to get into the submucosal layer LM. Thus, the electrode member 14 is led to as far as the submucosal layer LM across the mucosal layer LU to start a dissection of the diseased mucosal region D. In this state, the mucosa is incised along outer peripheral side of the cauterization spots B by moving the endoscopic insertion rod S and angularly turning its articular portion under observation through the endoscopic observation window W.

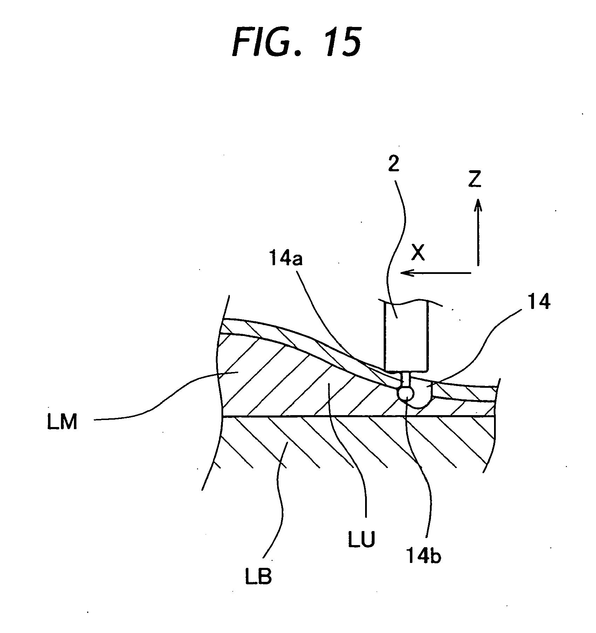

As shown in Fig. 15, the mucosal layer LU is dissected by pushing forward the rod portion 14a of the electrode member 14 along the mucosal layer LU while conducting a high frequency current through the electrode member 14. At this time, due to swinging motions of the flexible sheath 2, the tool may be disturbed by deviational forces acting in a direction away from the mucosal layer LU. In addition, the tool may be met by a force acting to pull the electrode member 14 into the flexible sheath 2. However, on such an occasion, a dissection treatment can be performed smoothly thanks to an anchor effect of the spherically bulged head portion 14b which is deeply embedded in the submucosal layer LM. Further, in case a dissection is made by advancing the electrode member 14 in the direction of arrow X in Fig. 15, the mucosal layer LU is incised by the rod portion 14a of the electrode member 14, which is arranged to grip the mucosal layer LU between its spherically bulged portion 14b and the annular flat end face P, which is formed jointly by end faces of the body tube 2a of the flexible tube 2 and the rigid tip member 20. Accordingly, the electrode member 14 is prevented from retracting in the direction of arrow Z. That is to say, a dissection can be made in a stabilized state. Of course, there is little possibility of the electrode member 14 easily getting out of the mucosal layer LU under the influence of pulsations or other movements on the part of the mucosal layer LU.

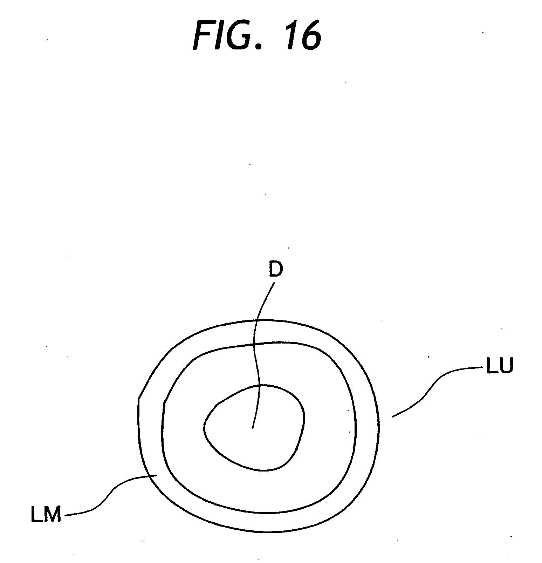

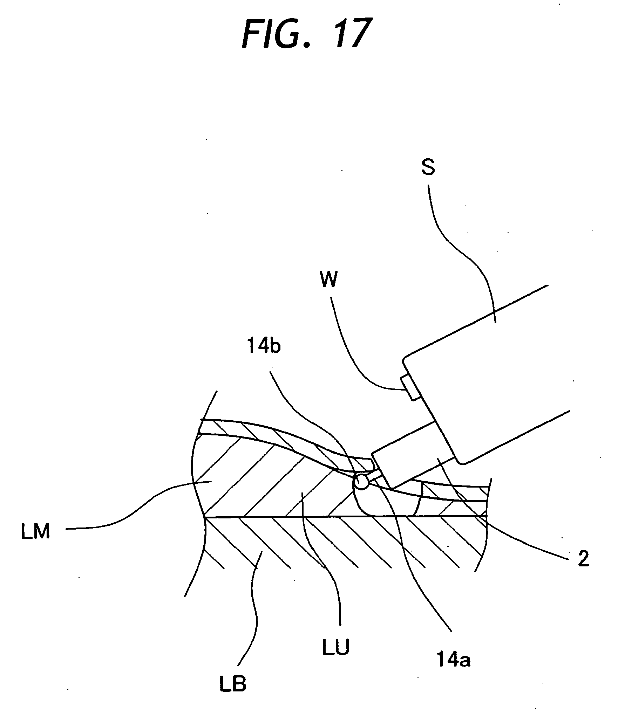

As described above, the mucosal layer LU is dissected all around the borders of the diseased mucosal region D as shown in Fig.16. However, at this point of time, the diseased mucosal region D cannot be removed because it is still connected with the muscle layer LB by way of the fibrous submucosal layer LM. That is to say, for removal of the diseased mucosal region LU, it is necessary to cut off connecting fibrous tissues. This can also be done by the use of the high frequency tool 1. Namely, as shown in Fig. 17, the electrode member 14 which is projected out of the flexible sheath 2 of the high frequency tool 1 is put into an end of the submucosal layer LM which is now exposed as a result of the circumventive dissection of the diseased mucosal region D, and then manipulated into a horizontal movement along or into sweeping movements to and fro across the submucosal layer LM to cut off the latter. This can be done easily, for example, by angularly flexing a fore end portion of the endoscopic insertion rod S. Thus, the high frequency tool can perform a mucosal excision treatment quickly in an efficient manner. The fore end of the electrode member 14 is terminated with the spherically bulged portion 14b which acts to lift up a submocosal layer LM when pushed into the latter, contributing to perform the mucosal excision treatment in an accelerated manner. In the course of the excision treatment, the spherically bulged end portion 14b of the electrode member 14 appears as an index point in picture images monitored through the endoscopic observation window W, helping perform an excision treatment in a safe manner.

Further, at the time of a mucosal excision treatment, it becomes necessary to supply physiological saline. Although the diseased mucosal region D was once swelled and elevated by a regional injection, it is usually the case that shrinkage occurs to the swelled and elevated portion due to exudation or absorption of injected physiological saline in the process of the dissecting treatment. Therefore, physiological saline is fed during an excision treatment thereby to maintain the submucosal layer LM in a swelled and elevated state. At this time, physiological saline can be supplied through the communication passages 23, while retracting the electrode member 14 to a position on the proximal side of the rigid tip member 22 and holding the annular end face P in abutting engagement with the submucosal layer LM. By so doing, physiological saline which is supplied via the connection port 3a of the connector pipe 3 and the flexible sheath 2 can be directly injected into the submucosal layer LM, permitting a more efficient supply of physiological saline. Accordingly, the submucosal layer LM can be maintained in a swelled and elevated state throughout an excision treatment.

In this manner, the additional supply of physiological saline can be made without necessitating interruption of an excision treatment for troublesome instrument replacements such as extraction of the high frequency tool 1 out of the endoscopic instrument insertion channel C and inserting a syringe in place of the extracted high frequency tool 1. Thus, considering this point alone, an operator is allowed to perform an excision treatment smoothly in a highly efficient manner. Besides, since nothing is projected from the annular end face P, the fore distal end of the groove 21 can be brought into abutting engagement with the submucosal layer LM and precisely directed toward a region which is in need of a supply of physiological saline. It follows that the submucosal layer LM can be maintained in a swelled and elevated state in an assured manner, helping finish a mucosal excision treatment in a quick and safe manner.

If necessary, a suction force can be applied to the mucosa LU. Suction drainage can be required not only during a mucosal excision treatment but also in pre- and post-treatment stages. Any way, a suction tube from an aspirator is connected to the connection port 3a of the connector pipe 3, and the aspirator is turned on and off, say, by means of a foot switch. Thus, in a stage in need of suction drainage, an operator can turn on an aspirator by the use of a foot switch thereby developing a vacuum pressure in a suction tube to start suction drainage through the communication passages 23.

By the way, during a mucosal excision treatment, there is a possibility of bleeding from a dissected portion. In such a case, physiological saline or the like is fed under high pressure into the flexible sheath 2 through the connection port 3a of the connector pipe 3. The supplied physiological saline is spurted out from the communication passages 23 which are defined internally of the rigid tip member 20 between the adjacent axial ridge portions 15 on the rod portion 14a of the electrode member 14, in communication with the connection port 3a. Accordingly, a bleeding site can be cleaned quickly by the physiological saline which is spurted out from the communication passages 23.

This irrigative cleaning can be performed irrespective of a mucosal excision treatment, namely, irrespective of the position of the electrode member 14 and even in the process of a mucosal excision treatment with the electrode member 14 projected out of the fore distal end the flexible sheath 2. Besides, when the whole high frequency knife 3 is retracted into a position on the proximal side of the rigid tip member 20 as shown in Fig. 5, the axial passage hole 21 of the rigid tip end 20 is totally opened up to spurt an irrigation liquid at a greater flow rate.

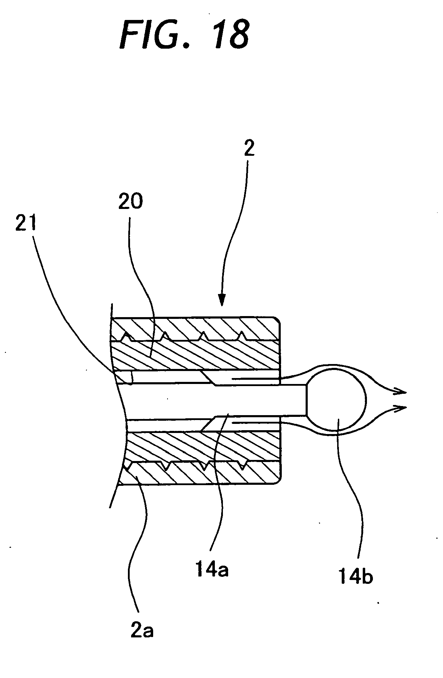

After an irrigative cleaning treatment of a bleeding portion, the spherically bulged portion 14b of the electrode member 14 is held in abutting engagement with the bleeding portion and in this state a high frequency current is conducted to stanch bleeding by a coagulative cauterizing treatment. At the time of this stanching treatment, the arcs of high frequency current which occur in radial directions of the spherically bulged portion 14b help stanch bleeding in a very efficiently manner. Fragments of tissues or blood may deposit on the spherically bulged end portion 14b as well as on the rod portion 14a of the electrode member 14 at the time of dissection or excision treatment or at the time of a stanching treatment. Deposition of tissue fragments and blood may hinder an effective high frequency current flow toward an aimed mucosal region or toward a bleeding portion from the electrode member 14. In such a case, the electrode member 14 can be cleaned by spurting out a cleaning liquid from the communication passages 23 toward the electrode member 14 which is projected out of the flexible sheath 2 while holding the spherically bulged end portion in a spaced position from the mucosa, as shown in Fig. 18. As indicated by arrows in the same figure, a cleaning liquid which is spurted out from the communication passages 23 is directed forward toward the spherically bulged portion 14b, forming scrubbing currents along and all around the rod portion 14a and spherically bulged portion 14b of the electrode member 14 to wash away deposited contaminants. Especially at the spherically bulged portion14b which is in a curved shape in its entirety, all of its surfaces are covered with currents of the spurted liquid and thereby completely cleaned to an almost speckless state.

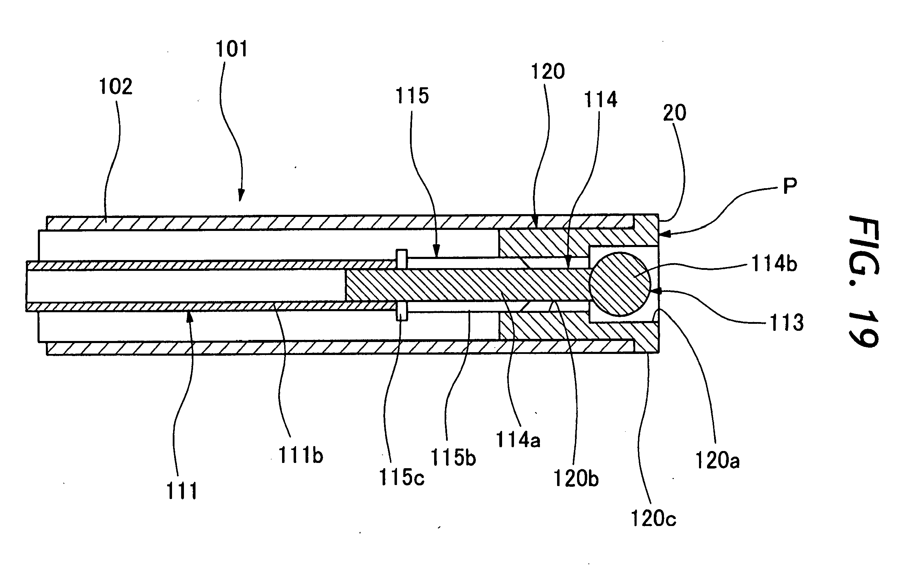

If desired, a high frequency tool 101 of Fig. 19 may be employed in place of the above-described high frequency tool 1. The high frequency tool 101 is composed of a flexible sheath 102, and a tubular rigid tip member 120 which is fixedly fitted at a fore distal end of the flexible sheath 102. The tubular rigid tip member 120 is of a stepped structure having a large diameter portion 120a and a small diameter portion 120b. Further, the tubular rigid tip member 120 is provided with a flange 120c around its fore distal end. Outer peripheral surface of the flange 120c is disposed flush with outer peripheral surface of the flexible sheath 102. The tubular rigid tip member 120 is formed of an electrically insulating material like ceramics, and formed with a plane annular end face P at its fore distal end of the high frequency tool 101 to be brought into abutting engagement with a mucosal layer.

An electrode member 114, to serve as a high frequency knife 113, is provided with a spherically bulged portion 114b at a fore distal end of a rod portion 114a. The spherically bulged portion 114b has an outside diameter which is larger than the small diameter portion 120b of the tubular rigid tip member 120 but smaller than the large diameter portion 120a of the latter. Thus, in comparison with the foregoing first embodiment, if the small diameter portion 120b is substantially same as the axial passage hole 21 in diameter, the spherically bulged portion 114b of the electrode member 114 of the high frequency knife 113 can be formed in a larger size than the spherically bulged portion 14b of the electrode member 14 in the foregoing embodiment. The large diameter portion 120a of the tubular rigid tip member 120 has a depth which is sufficient to receive and accommodate the spherically bulged portion 114b of the electrode member 114 completely when the latter is manipulated in a retracting direction.

Axial ribs or ridges 115 are formed around the rod portion 114a for the purpose of aligning the electrode member 114 with the center axis of the tubular rigid tip member 120 and at the same time for defining communication passages for supply of a liquid like physiological saline, for example. Preferably, four ridges (or three ridges) 115 are provided in equidistant radial positions around the outer periphery of the electrode member 114. Outer surfaces 115b of the ridges 115 are formed at such a height as to inscribe the inner periphery of the small diameter portion 120b of the tubular rigid tip member 120. Stoppers 115c are projected radially outward at proximal ends of the respective axial ridges 115. The stoppers 115c may be formed of a metallic material or an electrically insulating material like ceramics.

In assembling the high frequency tool 101 of the above construction, a hollow power supply wire 111b of a flexible cord 111 is passed through the flexible sheath 102 in the first place, and then the rod portion 114a of the electrode member 114 is placed into the small diameter portion 120b of the tubular rigid tip member 120 from the side of the large diameter portion120a. Thereafter, a rear end portion of the rod portion 114a is inserted into the hollow wire 111b and fixed in the latter, for example, by the use of a conductive adhesive agent. Finally, the tubular rigid tip member 120 is fitted into the fore distal end of the flexible sheath 102, fixing the flange 120c of the tubular rigid tip member 120 in abutting engagement with the fore distal end of the flexible tube 102 by the use of an adhesive agent. Thus, the high frequency tool 101 can be assembled very easily.

The spherically bulged portion 114b is used for elevating a mucosal layer at the time of an excision treatment and is brought into abutting engagement with a bleeding portion at the time of a stanching treatment. In order to serve for these purposes, it is desirable that the spherically bulged portion 114b in large enough in outside diameter. The spherically bulged portion 114b can be formed in a desired size insofar as it can be accommodated in the large diameter portion 120a of the tubular rigid tip member 120. In this regard, a stroke length of the manipulation means 4 can be shorted by forming the large diameter portion 120a in a minimum necessary depth for accommodating and keeping the spherically bulged portion 114b out of contact with walls in a body cavity.

Further, in this case, the spherically bulged portion is located forward of the stepped wall portion between the large and small diameter portions 120a and 120b of the tubular rigid tip member 120, so that it is possible to supply liquid like physiological saline even when the electrode member 114 is retracted into the rigid tip member 120. Even if the spherically bulged portion 114b is abutted against the stepped wall portion between the large and small diameter portion 120a and 120b, liquid supply passages can be providing grooves along the inner periphery of the small diameter portion 120b.

## Claims

1. A high frequency tool (1, 101) having a high frequency knife (13, 113) at a fore distal end of a flexible power supply cord (11, 111) for use in endoscopic electro surgery, wherein:
a flexible sheath (2, 102) comprises a body tube (2a) having a rigid tubular tip member (20, 120) fitted in a fore distal end portion in such a way as to form a flat annular end face (P) at a fore distal end of said body tube (2a):
said tubular rigid member (20, 120) is formed of an electrically insulating material, internally defining a through axial passage hole (21) leading to said fore distal end of said body tube (2a); and
said high frequency knife (13, 113) is constituted by an electrode member (14, 114) protrusibly fitted in said tubular rigid tip member (20, 120);
**characterized in that**
said electrode member (14, 114) is formed with a spherically bulged portion (14b, 114b) at a fore distal end of a rod portion (14a, 114a);
said spherically bulged portion (14b, 114b) has a diameter which is larger than the diameter of said rod portion (14a, 114a); and
said spherically bulged portion (14b, 114b) is adapted to retract fully into said axial passage hole (21) and to protrude fully out of said flat annular end face (P) through said axial passage hole (21).

2. A high frequency tool as set forth in claim 1, wherein an end face of said tubular rigid tip member (20) is disposed flush with an end face (20b) of said flexible sheath (2) to provide said flat annular end face (P) at said fore distal end of said flexible sheath (2).

3. A high frequency tool as set forth in claim 1 or 2, wherein said body tube (2a) is connectible to a liquid feed means to receive a supply of a liquid to be spurted into a body cavity from said fore distal end of said flexible sheath (2).

4. A high frequency tool as set forth in claim 3, wherein said tubular rigid tip member (20) is formed with a liquid supply passage or passages (23) as part of said axial passage hole (21), and said electrode member (14) is provided with a stopper (15c) to be brought into abutting engagement with said rigid tip member (20) to delimit a maximum protrusion length of said electrode member (14) from said flexible sheath (2).

5. A high frequency tool as set forth in claim 4, wherein said electrode member (14) is provided with axially extending ridge portions (15) at angular intervals around outer periphery of said rod portion (14a) and at a predetermined distance from said fore distal end with said spherically bulged portion (14b), said ridge portions (15) each having an outer surface located at an inscribing height relative to inner periphery of said axial passage hole (21) of said tubular rigid tip member (20) and being provided with a radial projection (15c) at a proximal end, said radial projection being extended radially outward beyond inner periphery of said tubular rigid tip member (20) to serve as said stopper, said electrode member (14) defining liquid feed passages between adjacent ridge portions (15) to intercommunicate inside and outside of said flexible sheath (2) when said stopper (15c) is brought into abutting engagement with a proximal end face of said tubular rigid tip member (20).

6. A high frequency tool as set forth in claim 5, wherein said electrode member (14) is retractable into said flexible sheath (2) to a position on the proximal side of said tubular rigid tip member (20), leaving said axial passage hole (21) of said tubular rigid tip member (20) in a fully opened state.

7. A high frequency tool as set forth in claim 5, wherein said electrode member (14) is arranged to have a protrusion length of 0.5mm to 4mm from a fore distal end of said tubular rigid tip member (20) when said stopper (15c) is brought into abutting engagement with the latter, a diameter of 0.4mm to 0.6mm in said rod portion (14a), and a maximum outside diameter of 0.8mm to 1.2mm at said spherically bulged portion (14b).

## Patentansprüche

1. Hochfrequenzwerkzeug (1, 101) mit einem Hochfrequenzmesser (13, 113) am vorderen freien Ende eines flexiblen Energiezuführkabels (11, 111) zur Verwendung bei der endoskopischen Elektrochirurgie, bei dem
eine flexible Hülle (2, 102) einen Schlauchkörper (2a) mit einem starren schlauchförmigen Vorderteil (20, 120) aufweist, der in einen vorderen freien Endbereich derart eingepasst ist, dass am vorderen freien Ende des Schlauchkörpers (2a) eine flache ringförmige Stirnfläche (P) gebildet ist;
wobei der schlauchförmige starre Teil (20, 120) aus einem Isolierstoff gebildet ist, im Inneren ein axiales Durchgangsloch (21) bildet, welches zu dem vorderen freien Ende des Schlauchkörpers (20a) führt; und
wobei das Hochfrequenzmesser (13, 113) gebildet wird durch ein Elektrodenelement (14, 114), welches ausfahrbar in das schlauchförmige starre Endteil (20, 120) eingesetzt ist,
**dadurch gekennzeichnet, dass**
das Elektrodenelement (14, 114) mit einem sphärisch ausgewölbten Abschnitt (14b, 114b) an einem vorderen freien Ende Stababschnitts (14a, 114a) gebildet ist;
der sphärisch ausgewölbte Abschnitt (14b, 114b) einen Durchmesser besitzt, der größer ist als der Durchmesser des Stababschnitts (14a, 114a); und
der sphärisch ausgewölbte Abschnitt (14b, 114b) dazu ausgebildet ist, vollständig in das axiale Durchgangsloch (21) zurückgezogen zu werden und durch das axiale Durchgangsloch (21) vollständig aus der flachen ringförmigen Stirnfläche (P) vorzustehen.

2. Hochfrequenzwerkzeug nach Anspruch 1, bei dem eine Stirnfläche des schlauchförmigen starren Endteils (20) bündig mit einer Stirnfläche (20b) der flexiblen Hülle (2) angeordnet ist, um die flache ringförmige Stirnfläche (P) an dem vorderen freien Ende der flexiblen Hülle (2) zu bilden.

3. Hochfrequenzwerkzeug nach Anspruch 1 oder 2, bei dem der Schlauchkörper (2a) mit einer Flüssigkeitszuführeinrichtung verbindbar ist, um eine Zufuhr einer Flüssigkeit aufzunehmen, die aus einem vorderen freien Ende der flexiblen Hülle (2) in einen Körperhohlraum einzuspülen ist.

4. Hochfrequenzwerkzeug nach Anspruch 3, bei dem der schlauchförmige starre Endteil (20) mit einem oder mehreren Flüssigkeitszuführkanälen (23) als Bestandteil des axialen Durchgangslochs (21) ausgebildet ist, und das Elektrodenelement (14) mit einem Anschlag (15c) versehen ist, der in Anlage an dem starren Vorderteil (20) zu bringen ist, um eine maximale Ausfahrlänge des Elektrodenelements (14) aus der flexiblen Hülle (2) zu begrenzen.

5. Hochfrequenzwerkzeug nach Anspruch 4, bei dem das Elektrodenelement (14) mit sich axial erstreckenden Rippenabschnitten (15) in Winkelintervallen über den Außenumfang des Stababschnitts (14a) und in einem vorbestimmten Abstand gegenüber dem vorderen freien Ende mit dem sphärisch ausgewölbten Abschnitt (14b) ausgestattet ist, wobei die Rippenabschnitte (15) jeweils eine Außenfläche aufweisen, die sich in einer eingeschriebenen Höhe bezüglich des Innenumfangs des axialen Durchgangslochs (21) des schlauchförmigen starren Endteils (20) befindet und an einem proximalen Ende mit einem Radialvorsprung (15a) versehen ist, der sich radial nach außen über den Innenumfang des schlauchförmigen starren Endteils (20) hinaus erstreckt, um den erwähnten Anschlag zu bilden, wobei das Elektrodenelement (14) zwischen benachbarten Rippenabschnitten (15) Flüssigkeitszuführkanäle definiert, um das Innere und das Äußere der flexiblen Hülle miteinander zu verbinden, wenn der Anschlag (15c) in Anlage mit einer proximalen Stirnfläche des schlauchförmigen starren Endteils (20) gebracht ist.

6. Hochfrequenzwerkzeug nach Anspruch 5, bei dem das Elektrodenelement (14) in die flexible Hülle zu einer Stelle auf der proximalen Seite des schlauchförmigen starren Endteils (20) rückziehbar ist, wobei das axiale Durchgangsloch (21) des schlauchförmigen starren Endteils (20) in einem vollständig geöffneten Zustand verbleibt.

7. Hochfrequenzwerkzeug nach Anspruch 5, bei dem das Elektrodenteil (14) derart ausgestaltet ist, dass es eine Ausfahrlänge von 0,5 mm bis 4 mm gegenüber einem vorderen distalen Ende des schlauchförmigen starren Endteils (20) hat, wenn der Anschlag (15c) in Anlage mit letzterem gebracht ist, einen Durchmesser von 0,4 mm bis 0,6 mm in dem Stababschnitt (14a) aufweist, und einen maximalen Außendurchmesser von 0,8 mm bis 1,2 mm an dem sphärisch ausgewölbten Abschnitt (14b) besitzt.

## Revendications

1. Instrument à haute fréquence (1, 101) comprenant un couteau haute fréquence (13, 113) à une extrémité distale antérieure d'un cordon d'alimentation électrique souple (11, 111) pour un usage en électrochirurgie endoscopique, dans lequel :
une gaine souple (2, 102) comprend un corps en forme de tube (2a) ayant un organe formant pointe tubulaire rigide (20, 120) monté dans une partie d'extrémité distale antérieure de sorte à former une face d'extrémité annulaire plate (P) à une extrémité distale antérieure dudit corps en forme de tube (2a) ;
ledit organe formant pointe tubulaire rigide (20, 120) est réalisé en un matériau électriquement isolant définissant en interne un trou de passage traversant axial (21) conduisant à ladite extrémité distale antérieure dudit corps en forme de tube (2a) ; et
ledit couteau haute fréquence (13, 113) est constitué par un organe formant électrode (14, 114) monté de manière protubérante dans ledit organe formant pointe tubulaire rigide (20, 120) ;
**caractérisé en ce que**
ledit organe formant électrode (14, 114) comporte une partie sphériquement bombée (14b, 114b) à une extrémité distale antérieure d'une partie formant tige (14a, 114a) ;
ladite partie sphériquement bombée (14b, 114b) a un diamètre qui est plus large que le diamètre de ladite partie formant tige (14a, 114a) ; et
ladite partie sphériquement bombée (14b, 114b) est adaptée pour se rétracter complètement à l'intérieur dudit trou de passage traversant axial (21) et pour se projeter en saillie complètement à l'extérieur de ladite face d'extrémité annulaire plate (P) au travers dudit trou de passage traversant axial (21).

2. Instrument à haute fréquence selon la revendication 1, dans lequel une face d'extrémité dudit organe formant pointe tubulaire rigide (20) affleure une face d'extrémité (20b) de ladite gaine souple (2) de sorte à présenter ladite face d'extrémité annulaire plate (P) à ladite extrémité distale antérieure de ladite gaine souple (2).

3. Instrument à haute fréquence selon la revendication 1 ou 2, dans lequel ledit corps en forme de tube (2a) peut être raccordé à des moyens d'alimentation en liquide pour recevoir une fourniture d'un liquide devant être injecté dans une cavité corporelle à partir de ladite extrémité distale antérieure de ladite gaine souple (2).

4. Instrument à haute fréquence selon la revendication 3, dans lequel ledit organe formant pointe tubulaire rigide (20) comporte un ou plusieurs passages d'alimentation en liquide (23) faisant partie dudit trou de passage traversant axial (21), et ledit organe formant électrode (14) comporte un arrêtoir (15c) devant être amené en butée et en prise avec ledit organe formant pointe tubulaire rigide (20) de sorte à délimiter une longueur de saillie maximum dudit organe formant électrode (14) par rapport à ladite gaine souple (2).

5. Instrument à haute fréquence selon la revendication 4, dans lequel ledit organe formant électrode (14) comporte des parties formant arête (15) s'étendant axialement à intervalles réguliers autour d'une périphérie extérieure de ladite partie formant tige (14a) et à une distance prédéterminée de ladite extrémité distale antérieure par rapport à ladite partie sphériquement bombée (14b), lesdites parties formant arête (15) ayant chacune une surface extérieure située à une hauteur inscrite par rapport à une périphérie intérieure dudit trou de passage traversant axial (21) dudit organe formant pointe tubulaire rigide (20) et comportant une protubérance radiale (15c) à une extrémité proximale, ladite protubérance radiale s'étendant radialement à l'extérieur au-delà d'une périphérie intérieure dudit organe formant pointe tubulaire rigide (20) de sorte à jouer le rôle dudit arrêtoir, ledit organe formant électrode (14) définissant des passages d'alimentation en liquide entre des parties formant arête (15) adjacentes pour intercommuniquer à l'intérieur et à l'extérieur de ladite gaine souple (2) lorsque ledit arrêtoir (15c) est amené en butée et en prise avec une face d'extrémité proximale dudit organe formant pointe tubulaire rigide (20).

6. Instrument à haute fréquence selon la revendication 5, dans lequel ledit organe formant électrode (14) peut se rétracter à l'intérieur de ladite gaine souple (2) à une position sur le côté proximal dudit organe formant pointe tubulaire rigide (20), laissant ledit trou de passage traversant axial (21) dudit organe formant pointe tubulaire rigide (20) dans un état complètement ouvert.

7. Instrument à haute fréquence selon la revendication 5, dans lequel ledit organe formant électrode (14) est configuré de sorte à avoir une longueur de saillie de 0,5 mm à 4 mm à partir d'une extrémité distale antérieure dudit organe formant pointe tubulaire rigide (20) lorsque ledit arrêtoir (15c) est amené en butée et en prise avec cette dernière, un diamètre de 0,4 mm à 0,6 mm dans lesdites parties formant tige (14a), et un diamètre extérieur maximum de 0,8 mm à 1,2 mm au niveau de ladite partie sphériquement bombée (14b).
